(19) Europäisches Patentamt
European Patent Office
Office européen des brevets

(11) **EP 2 058 335 B1**

(12) **EUROPEAN PATENT SPECIFICATION**

(45) Date of publication and mention
of the grant of the patent:
**24.06.2020 Bulletin 2020/26**

(21) Application number: **07021661.9**

(22) Date of filing: **07.11.2007**

(51) Int Cl.:
*C07K 17/14* *(2006.01)*       *C07K 17/02* *(2006.01)*
*G01N 33/543* *(2006.01)*      *C12N 11/02* *(2006.01)*
*C12N 11/14* *(2006.01)*       *C12Q 1/68* *(2018.01)*

(54) **Biocompatible three dimensional matrix for the immobilization of biological substances**

Biologisch verträgliche, dreidimensionale Matrix zur Immobilisierung von biologischen Substanzen

Matrice tridimensionnelle biocompatible pour l'immobilisation de substances biologiques

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**13.05.2009 Bulletin 2009/20**

(73) Proprietor: **LEUKOCARE AG**
**82152 Martinsried / München (DE)**

(72) Inventors:
• **Scholz, Martin**
**61440 Oberursel (DE)**
• **Margraf, Stefan**
**60318 Frankfurt/Main (DE)**
• **Altrichter, Jens**
**18196 Kavelstorf (DE)**

(74) Representative: **Vossius & Partner**
**Patentanwälte Rechtsanwälte mbB**
**Siebertstrasse 3**
**81675 München (DE)**

(56) References cited:
EP-A- 1 571 204       EP-A- 1 852 443
WO-A-01/02858       WO-A-01/06011
WO-A-01/14425       WO-A-02/46756
WO-A1-96/40297       WO-A1-97/17436
WO-A1-03/030949       US-A- 5 760 130

• MANDENIUS C F ET AL: "THE INTERACTION OF PROTEINS AND CELLS WITH AFFINITY LIGANDS COVALENTLY COUPLED TO SILICON SURFACES AS MONITORED BY ELLIPSOMETRY" ANALYTICAL BIOCHEMISTRY, ACADEMIC PRESS, NEW YORK, NY, US, vol. 137, no. 1, 1984, pages 106-114, XP008069229 ISSN: 0003-2697
• PIEHLER J ET AL: "A high-density poly(ethylene glycol) polymer brush for immobilisation on glass-type surfaces" BIOSENSORS & BIOELECTRONICS, ELSEVIER SCIENCE PUBLISHERS, BARKING, GB, vol. 15, no. 9-10, November 2000 (2000-11), pages 473-481, XP002400094 ISSN: 0956-5663
• STADTMAN ER ET AL: "Free radical-mediated oxidation of free amino acids and amino acid residues in proteins", AMINO ACIDS, vol. 25, 29 July 2003 (2003-07-29), pages 207-218,
• GONZALEZ M ET AL: "Extremely high thermal stability of streptavdin and avidin upon biotin binding", BIOMOLECULAR ENGINEERING, vol. 16, 1 January 1999 (1999-01-01), pages 67-72,
• VERMEER A W P ET AL: "The Thermal Stability of Immunoglobulin: Unfolding and Aggregation of a Multi-Domain Protein", BIOPHYSICAL JOURNAL, vol. 78, 1 January 2000 (2000-01-01), pages 394-404,

Remarks:
The file contains technical information submitted after the application was filed and not included in this specification

## Description

The present invention is defined by the claims. Generally described herein is a method of producing a solid coated carrier carrying biological material. Furthermore, described herein is a solid coated carrier to which biological material is attached and uses of the solid coated carrier for the preparation of a medical product. Moreover, described herein is a method for the contacting, filtration or cleaning of blood, lymph or liquor cerebrospinalis or parts thereof of a patient, a method for the diagnosis of a disease and a diagnostic composition.

The systemic application e.g. of monoclonal antibodies or other molecules is an important and growing field for the treatment of tumor patients, metabolic disorders, immune diseases or other diseases. However, the systemic application of these molecules is extremely expensive and is frequently associated with severe side effects and increased mortality. Moreover, the fate of the injected biologicals is often ill defined and severe side effects can not be foreseen in the individual patient. WO 03/030949 discloses the protective effects of stabilizers on gamma irradiated immobilized anti-insulin monoclonal antibodies.

An option for the application of novel therapeutic procedures is the use of (medical) devices with immobilized antibodies. This procedure has the advantage over the standard therapies to use the functional properties of the antibody without the possibly detrimental effects of a systemic application. Therefore, severe side effects frequently observed using antibodies systemically are not seen using this safe procedure based on a (medical) device. A transient or permanent application of such a device within the blood stream aiming to manipulate the physiology, the immune system, immune cells or other constituents of the blood, or floating tumor cells, with immobilized, highly defined quantities of biological material on a defined surface, which could e.g. imply a defined density of the biological material within a defined volume would circumvent the heavy burden of systemic application of certain drugs.

Therapies are described in the art in which TNFα is inactivated by the use of inactivating antibodies like Remicade®. However, the simple administration of such antibodies to a patient is known to be associated with severe, even deadly, side-effects (blindness, shock, allergic reaction and the like). Moreover, the administration of full antibodies or fragments or derivatives thereof requires a humanization and/or other modifications of the antibodies in order to reduce or circumvent an inactivation of the antibodies by an immune reaction of the human patient against the antibodies.

A further approach described in the art is known under the name "TheraSorb®" (Miltenyi Biotech GmbH, Bergisch-Gladbach, Germany); see R. Bambauer et al., Ther Apher Dial. 2003, 7(4):382-90 and G. Wallukat et al., International Journal of Cardiology, 1996, 54 (2):191-195. TheraSorb® is used to remove LDL-cholesterol, immunoglobulins, immune complexes, antibody fragments or fibrinogen. The apheresis columns used in this method contain sepharose with coupled polyclonal antibodies. The procedure requires cell separation to deliver plasma to the column. This is a major disadvantage of the system, because this is an extravagant expenditure. The flow rate is low and therefore only small amounts of plasma are treated. The system is also very expensive due to its complexity.

The above discussion demonstrates that the methods and systems available so far for the treatment of the recited diseases are either associated with severe side effects or rather burdensome to implement. Thus, the technical problem underlying the present invention was to provide means and methods which enable the treatment of patients with biological material such as cells and proteins, which improve this situation. The solution to this technical problem is achieved by the embodiments characterized in the claims.

Accordingly, the present invention provides in a first embodiment a method of producing a solid coated carrier carrying an antibody, consisting of the steps of: (a) incubating a solid carrier with a solution comprising 0.1 to 10 % (w/w) or (v/v) of at least one suitable coupler, wherein the coupler is selected from diaminoalkanes, dicarboxylic acids or bi- or multi functionalized polyethyleneglycol, and subsequently removing the solution, thereby attaching a layer of the at least one suitable coupler to the carrier, and, optionally, further comprising a step (a') drying the carrier until the residual content of the solution is less than 10 % of the originally applied solution; (b) attaching the antibody to the layer of the at least one suitable coupler of step (a) attached to the carrier by incubating the carrier with a preferably buffered aqueous solution containing the antibody and subsequently removing the aqueous solution, further comprising a step (b') subsequent to the step (b) and previous to step (c): (b') incubating the carrier in a buffered aqueous solution containing a blocking agent and removing the aqueous solution; or optionally further comprising a step (b") subsequent to the step (b) and previous to step (c): (b") blocking unbound binding sites using an aqueous solution containing 0.5-10 % (w/w) substances selected from the group consisting of (poly)peptides, hydroxyethylstarch (HES), mannitol, sorbitol and polyethyleneglycol (PEG), milk, soya, wheat or egg derived protein and optionally performing one or more washing steps using an aqueous solution after blocking; (c) incubating the carrier in an aqueous solution comprising one or more substances selected from polypeptides, at least one amino acid selected from the group consisting of glycine, aspartic acid, glutamic acid, proline, arginine, alanine, asparagine, aspartic acid, glutamic acid, glutamine, ornithine, hydroxyproline, citrulline, inuline, lysine, leucine, isoleucine, serine, valine, threonine, mannitol, or starch or a mixture thereof, such that the antibody is partially or completely covered by said one or more substances; and, optionally, air-drying the carrier until the residual water content is < 20 % (w/w); wherein steps (a), (b) and (c) are carried out in the above described order; and (d) sterilizing the produced solid coated carrier.

Also described herein is a method of producing a solid coated carrier carrying biological material, comprising the steps of:

(a) incubating a solid carrier with a solution comprising 0.1 to 10 % (w/w) or (v/v) of at least one silane or another suitable coupler and subsequently removing the solution;
(b) attaching the biological material to the carrier by incubating the carrier with a preferably buffered aqueous solution containing the biological material and subsequently removing the aqueous solution; and
(c) incubating the carrier in (i) an aqueous solution comprising one or more substances selected from (poly)peptides, amino acids, starch, sugars, phosphates, polyalcohols, polyethyleneglycols (PEGs) or a mixture thereof, (ii) an ionic liquid or (iii) compatible solutes, or a mixture thereof, such that the biological material is partially or completely covered by said one or more substances.

Steps (a), (b) and (c) are carried out in the above described order.

The term "solid carrier" defines in the context of the disclosure a carrier of solid material. The material of the carrier may be either of compact or porous structure. As described herein below, it is preferred that the carrier is of a material selected from the group consisting of glass, polyurethane, polyester, polysulfone, polyethylene, polypropylene, polyacryl, polyacrylnitril, polyamid, PMMA, fleece wadding, open porous foam plastic or glass and reticular plastic or glass and structures derived from marine sponges (porifera).

The term "preferably buffered" according to the present disclosure refers to a solution which is preferably buffered.

The term "biological material" describes in the context of the disclosure material isolated from living or dead organisms or parts thereof or producible in artificial biological systems and chemically modified derivatives thereof. An example for an artificial biological system comprises means for the in vitro synthesis of nucleic acid molecules or (poly)peptides or the production by recombinant DNA technologies. As described in detail herein below examples for such biological material comprise eukaryotic cells, fragments of eukaryotic cells, prokaryotes, fragments of prokaryotes, archaebacteria, fragments of archaebacteria, viruses and viral fragments. The recited fragments of eukaryotic cells, prokaryotes, archaebacteria and viruses comprise one or more (poly)peptides, oligonucleotides, polynucleotides and polysaccharides or any combination thereof. (Poly)peptides, oligonucleotides, polynucleotides and polysaccharides are alternatively and still encompassed by the term biological material, if they do not find a counterpart in nature and are, e.g. (semi)synthetically or recombinantly produced (see below). In accordance with the invention as defined by the claims the biological material is an antibody.

The term "(poly)peptide" as used herein describes a group of molecules which comprise the group of peptides, as well as the group of polypeptides. The group of peptides consists of molecules up to 30 amino acids, the group of polypeptides consists of molecules with more than 30 amino acids. In accordance with the disclosure, the group of "polypeptides" comprises "proteins". Also in line with the definition the term "(poly)peptide" describes fragments of proteins. The definition of the term further comprises polypeptides which are dimers, trimers and higher oligomers, i.e. consisting of more than one string of amino acid molecules (chains of linked amino acids). As noted herein above, proteins are also understood as fragments of eukaryotic cells, prokaryotes, archaebacteria and viruses in case said proteins are isolated from eukaryotic cells, prokaryotes, archaebacteria and viruses. The term also applies to (poly)peptides which are produced by recombinant DNA technologies or other technologies employing e.g. cells for the production of the recited compounds.

The term "oligonucleotides" describes in the context of the disclosure nucleic acid molecules consisting of at least ten and up to 30 nucleotides. The term "polynucleotides" describes nucleic acid molecules consisting of more than 30 nucleotides. Oligonucleotides and polynucleotides described herein may be DNA, RNA, PNA and the like. The group of molecules subsumed under polynucleotides also comprise complete genes or chromosomes and fragments thereof. Also included by said definition are vectors such as cloning and expression vectors.

The term "polysaccharide" defines in the context of the disclosure polymers consisting of two or more than two saccharides and thus includes molecules otherwise known in the art as oligosaccharides. Said polysaccharides may consist of chains of branched or non-branched saccharides.

The term "silane" is used in the context of the disclosure in line with the definition of the International Union of Pure and Applied Chemistry (IUPAC) and describes a group of compounds consisting of a silicon matrix and hydrogen. A silane according to this definition may be branched (iso- and neo-silanes) or non-branched (n-silanes).

Suitable coupler or coupling reagents are bi- or multi functionalized organic molecules like diaminoalkanes e.g. 1,6 diaminohexane, dicarboxylic acids e.g. 1,6 hexanedioic acid, or polyethyleneglycol, functionalized by e.g. cyano-, amino or carbogyl groups.

The term "body fluid" defines in the context of the disclosure fluids derivable in samples from a patient, preferably a human patient. Examples for such body fluids comprise blood, lymph or liquor cerebrospinal or parts thereof, e.g. blood plasma or liquid fractions comprising albumin, as well as enriched leukocytes (e.g. from leukapheresis).

The term "patient" as used throughout the present disclosure comprises ill or diseased as well as healthy subjects. A patient in accordance with the present disclosure is any person who receives medical attention, care, or treatment. The person is most often but not always ill or injured and, if so, in need of treatment by a physician or other medical professional. In other terms, the term "patient" is interchangeably used with "subject" which may or may not be ill. The subject can be an animal, preferably a mammal, most preferably a human. In accordance with the above, a patient is also, for example, a healthy human who is, on an acute or routine basis, diagnosed for a disease or health status. In other terms, the disclosure may be used to find out whether a patient suffers from a certain disease (or a combination of diseases), is prone to develop such a disease or combination thereof or not.

The removal of the solutions is understood as a qualitative removal of the solution. Thus, the solution is removed at least to a degree were the remaining solution does not significantly change the quality of a solution used in a subsequent step of the method of the disclosure. The removal may be effected e.g. by suction, e.g. by using a conventional pump to which a pipette may be attached, and the like, compression or blowing. Optionally, such steps are combined and may be further combined with air drying. Preferably, the solution is removed in volume to at least 95 % such as at least 98 % or at least 99 %, 99.5 % or 99.8 %.

The term "incubating" refers to an incubation under conditions that allows the compounds recited in steps (a) to (c) to attach to the carrier or to a layer of molecules as mentioned in steps (a) and/or (b) attached to the carrier, optionally via an indirect binding. Incubation conditions include those where incubations are effected from 20 minutes to 12 hours depending on the step and the temperature. In general, antibodies are incubated for 1 hour at 37 °C, which is the maximum temperature where IgM antibodies are stable. IgG antibodies can be incubated to up to 50 °C. The temperature range may be from 4 °C to 50 °C, preferably 20 to 37 °C, depending on the step and the incubation time. It is preferred that, in step (a), incubation is effected for a period of 20 minutes at room temperature, in step (b) for 1 hour at 37 °C and in step (c) for 1 hour at room temperature. It is understood that in order to expedite the procedure or to improve the result, the incubation times and temperatures can be varied according to the substances used in the incubation. For example, if methanol is used as solvent in step (a), the temperature should be kept low at around 20 °C, e.g. from about 15 to about 25 °C, in order not to let the methanol evaporate.

The skilled artisan is aware that the term "room temperature" can imply different temperatures depending on the location and the outside temperature. It usually ranges between 17 and 23 °C.

The solution recited in step (a) of the method of the disclosure may be an aqueous or a non-aqueous solution. An aqueous solution in step (a) according to the method of the disclosure may, in addition to the silane, comprise diluted proteins and further diluted components such as sugars or alcohols. A preferred protein in this context is albumin. Albumin, in accordance with the present disclosure comprises albumins from animals, preferably mammals, more preferably from humans, and from plant seeds. Albumins form the major constituent of serum in mammals. Further examples of albumins according to the present disclosure are $\alpha$-lactalbumin and ovalbumin in animals, legumelin from peas, leucosin from wheat, rye or barley or legumelin from legumes. In case of potential allergens, it is preferred that the albumin is hydrolyzed, especially to enhance the compatibility with the human or other animal body. The same holds true for the non-aqueous solution in an alternative embodiment of step (a). In the context of the present disclosure, the term "aqueous solvent" is not limited to (but includes) $H_2O$ but extended to hydrophilic solvents mixable with water thus able to form a uniform phase.

Examples for aqueous solvents comprise, but are not limited to $H_2O$, methanol, ethanol or higher alcohols or mixtures thereof. Examples for non-aqueous solvents comprise, but are not limited to dimethylsulfoxide (DMSO), benzene, toluene, xylene and ethylbenzene, or aliphatic solvents like pentane, hexane, heptane or mixtures thereof.

It is preferred that the solvent of the solution in step (a) is $H_2O$, methanol, ethanol or mixtures thereof, dimetylsulfoxide (DMSO), benzene, toluene, xylene and ethylbenzene or pentane, hexane, heptane or mixtures thereof. The most preferred solvent of the solution of step (a) is methanol or ethanol. Correspondingly, a solution means a solution comprising a solvent and an aqueous solution means a solution comprising a hydrophilic solvent.

According to the disclosure the concentration of the at least one silane in step (a) of the method is in a range between 0.1 and 10 % (w/w) or (v/v), wherein this range simultaneously defines the concentration of all silanes in the solution. In other terms, the contribution of all silanes together, if more than one silane is employed, is in the range of 0.1 to 10 % (w/w) or (v/v). Preferably, the concentration is in a range of 0.5 to 5 % (w/w) or (v/v).

The attachment of the biological material to the solid carrier according to step (b) of the method of the disclosure is understood as a fixation of the material on the carrier. The fixation may be effected via a direct binding of the biological material to the solid carrier. Alternatively, the fixation may be effected via an indirect binding via a third compound such as a layer of one or more silanes covering the solid carrier. The term "covering" according to the present disclosure comprises full coverage as well as partial coverage of the solid carrier. In both alternatives the binding can be a binding via a covalent or a non-covalent bond. In line with the disclosure a covalent bond can be achieved e.g. by a chemical reaction between the biological material and a carrier material or between the silane (coating the carrier material) and the biological material. In the latter case the silane acts like a molecular bridge. Examples for non-covalent binding comprise weak bonds such as van-der-Waal's bonds or other polar bonds. Such non-covalent bonds occur e.g. between (poly)peptides and a solid carrier with a polyethylene surface.

Preferably the aqueous solution for the attachment of biological material such as e.g. antibodies is buffered and of low salt content. A low salt content according to the present disclosure is defined as a salt concentration of 0.9 % (w/w) or less, preferably less than 0.2 % (w/w). Generally, but not exclusively for the type IgG and IgY a more basic buffer, e. g. made of 15mM sodium carbonate and 35mM sodium hydrogencarbonate in water (pH 9) is useful whereas that for the attachment of IgM a more neutral buffer (pH 7.0 to 7.4), e.g. a phosphate buffer like PBS, is favorable.

Due to the incubation of the carrier to which the biological material in step (b) is attached in an aqueous solution according to step (c) of the method of the disclosure the biological material is embedded in a coating layer/coating matrix. By the embedding the accessible surface of the biological material is minimized. This means that the accessible surface is partially or completely covered . "Completely covered" as used throughout the present disclosure means that all possible binding sites on the accessible surface of the biological material are occupied/covered. In the case of nonspecific binding, the molecules completely covering the biological material bind unspecifically. Accordingly, in case of partial covering, only a part of the specific or unspecific binding sites of the accessible surface is covered. As a consequence of partial or complete coverage, the biological material is not accessible to other materials as well as non-material influences such as radiation any more or the accessibility for both is at least reduced.

The aqueous solution of step (c) preferably is of low salt content as defined above. It can optionally be buffered.

The steps of the method of the disclosure may be affected in batches in which the respective solutions are exchanged.

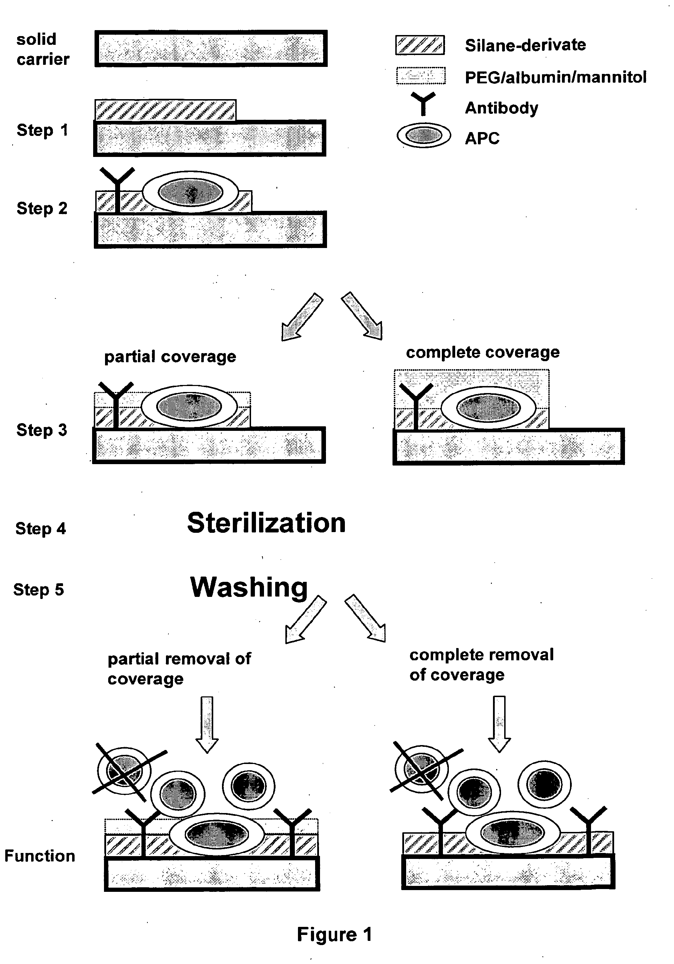

The method of the present disclosure provides solid carriers, which are coated with a matrix, into which the recited biological material is embedded. Examples of such a solid coated carrier comprising embedded biological material envisaged in the present disclosure and two preferred examples of the methods of the present disclosure are exemplified in the cross-section depicted in the scheme of figure 1.

Solid carriers produced by the method of the disclosure can be used in therapy and diagnosis.

Such therapies comprise the detoxification of body fluids from toxins such as dioxin, botulism toxin, tetanus toxin, LPS, septic poisons etc. The carriers may also be useful in the treatment of bacterial or viral diseases. One envisaged alternative application of the carriers produced by the method of the disclosure is the use in a therapy which requires the stimulation, elimination or removal of certain cells of a patient. An elimination of a specific population of cells may be e.g. indicated in the treatment of proliferative diseases (e.g. general in the treatment of cancer and particularly in the treatment of minimal residual cancer), autoimmune diseases or a treatment of diseases followed by organ transplantation. A stimulation of a specific population of cells may be envisaged e.g. in the treatment of a disease which is effected by the activation of cells of the immune system which are suitable, after stimulation, to eliminate a specific population of diseased cells. A further therapy approach makes use of solid coated carriers into which genetically or otherwise manipulated (e.g. differentiated by incubation with synthetic or natural compounds like cytokines, butyric acid, phorbol myristate acetate or all-trans-retinoic acid or physical manipulation like heat shock, cryopreservation, centrifugation) recombinant donor cells or non-manipulated donor cells are embedded. The embedded cells may secrete compounds such as hormones which reduce or heal endocrinal or metabolic disorders. Examples for the above described therapies are described in detail herein below. The therapy approaches may comprise an in vivo as well as an ex vivo application of the carrier produced by the method of the disclosure in the context of a (medical) device. Accordingly, a device comprising the solid coated carrier can be implanted into a patient for an in vivo application. For an ex vivo application a device comprising the solid coated carrier can be connected with the circulation of the body liquid to be treated. Blood derived from an artery or a vein of a patient may be e.g. led through such device and subsequently piped back into the patient (connection with the blood stream). Alternatively, samples of a body fluid may be incubated with the carrier in vitro. In a subsequent step of the latter treatment the body fluid can be reintroduced into the body of a patient.

Diagnostic applications of the solid carriers produced by the method of the disclosure are e.g. diagnostic methods which require the detection of toxins or specific cell populations in a body fluid. A diagnostic application using a solid coated carrier produced in accordance with the disclosure can be useful in a quantitative detection of toxins or specific cell populations. In such applications carriers may be employed to which a single type of biological material such as an antibody is attached. An incubation of such carrier with a defined volume of the body fluid from a patient will allow to quantitatively extrapolate an/the amount of the compound on the complete body of the patient after the analysis of the amount of the compound detected by the attached biological material in the sample. A qualitative detection of toxins or specific cell populations can be effected e.g. by the use of carriers to which many different types of biological materials are attached to. After incubation of the solid coated carrier with a body fluid from a patient the person skilled in the art can analyze which toxins or cell populations are specifically bound/detected by the attached biological material in the sample.

In the above described therapy approaches the embedded biological material will have a therapeutic impact e.g. on the blood, the lymph or the liquor without releasing the attached/immobilized biological material into the body fluid due to the fixation of the biological material on the solid carrier. It is preferred that no significant amount of the attached/immobilized biological material will be released into the body fluid. Thus, the required amount of a therapeutic or a diagnostic biological material is minimized. Due to the immobilization of the therapeutic or diagnostic biological material pharmacokinetic problems caused by circulating active biological material are also minimized.

The method of the disclosure allows the production of carriers with a clearly defined density of the biological material embedded on the surface of the carrier. Accordingly, the efficiency of the biological material embedded into the matrix of the produced carrier can be clearly and reproducibly defined. Moreover, a therapy which makes use of the carriers produced according to the method of the disclosure allows a defined onset of a therapy, e.g. by connecting the (medical) device comprising the carrier with the circulation of a body fluid, and a defined termination of the therapy, e.g. by disconnecting the device from the circulation of the body fluid.

A further superior feature of the carrier produced by the method of the disclosure is the improvement of the stability (shelf life) of the embedded biological material compared to conventionally produced material. Whereas the applicant does not wish to be bound by any theory it is believed that this improvement is achieved by the provision of the coating matrix which reduces the accessible surface of the biological material by partially or completely covering its surface which would otherwise be accessible for degenerative processes. According to this understanding the embedded biological material is protected and supported in its structure by the surrounding coating matrix. For example is the degradation or decomposition of antibodies coated to the carrier of the disclosure delayed or inhibited since the second layer applied to the carrier after coating the antibodies is protecting and stabilizing them.

Another superior feature of the method of the present disclosure and the produced coated carrier is the fact that the coating layer applied to the carrier in step (c) of the method of the disclosure enables for the sterilization of the produced carrier. Thus, for the first time, the present disclosure discloses a method of producing a solid coated carrier, coated with an, in general, comparably unstable and labile biological material, which can be sterilized and thus be applied in the treatment of patients or in methods where a sterile environment is needed. The second layer or coating matrix may well prevent the biological material coated to the carrier from degradation due to β-, γ- or X-ray-radiation in addition to enhancing its stability, as discussed above and shown in the appended examples.

The method being further described herein may comprise a step (d) after step (c): (d) drying the carrier until the residual water content is < 20% (w/w).

Accordingly, the residual water content may be calculated in a manner as known for the determination of the water content of wood. In the case of wood, the percentage of water content of wood (x) is determined by the calculation of the ratio between the mass of the water in the sample ($m_w$) and the mass of the water containing wood sample ($m_u$), multiplied with 100. The mass of water in the sample ($m_w$) can be determined by subtraction of the mass of the sample after its desiccation from the mass of the water containing wood sample ($m_u$). Accordingly, the percentage of water content of wood is calculated by the following formula:

$$X\ (\%) = \frac{m_w}{m_u} * 100$$

The residual water content of a preparation of carriers can be determined in analogy, wherein $m_w$ is the mass of the water in the sample of carriers and $m_u$ is the mass of the sample of carriers after the complete desiccation of the sample. In case of a spongiform carrier, $m_w$ is determined after squeezing the excess water out of the pores.

The method being further described herein may comprise a step (a') after step (a): (a') drying the carrier until the residual content of the solution is less than 10 %, preferably less than 5%, more preferably less than 2% such as less than 1 %, 0.5 % or 0.2 % of the originally applied solution. Preferred drying methods are described above. It is most preferred that drying is effected by air-drying.

The method being further described herein may comprise a step (b') subsequent to the step (b) and previous to step (c):
(b') incubating the carrier in a buffered aqueous solution containing a blocking agent and removing the aqueous solution.

A blocking agent may be used in the method for the production of a solid carrier in order to prevent unspecific binding of material added in subsequent steps of the method. This blocking may also have a positive effect on the conformation stability of the biological material before the incubation of the carrier in an aqueous solution according to step (c).

Blocking agents in line with the present disclosure comprise but are not limited to human or animal serum and proteins of such sera, (e.g. albumin), milk, egg proteins, plant derived proteins (e.g. soya, wheat) including hydrolysates of said proteins (e.g. gelatin, collagen). The buffered aqueous solution containing said blocking agent may also comprise amino acids (preferably glycine, aspartic and/or glutamic acids, proline, arginine, alanine, asparagine, aspartic acid, glutamic acid, glutamine, carnitine, ornithine, hydroxyproline, cysteine, homocysteine, citrulline, inuline, phenylanine, lysine, leucine, isoleucine, histidine, methionine, serine, valine, tyrosine, threonine, tryptophan) or derivates of amino acids (e.g. n-acetyl-tryptophan, beta-alanine, melanin, DOPA), sugars (preferably glucose, saccharose, sucrose), poly alcohols (preferably sorbitol, mannitol, glycerol, xylitol), polyethyleneglycol (PEG), hydroxyethylstarch (HES), phosphates (e.g. sodiummonodihydrogenphosphate, disodiumhydrogenphosphate), amphiphilic substances (preferably detergents like polysorbate, Triton X-100, buffers (preferably TRIS, HEPES).

Preferably the aqueous solution comprising the blocking agent is buffered and of low salt content as defined above. An example for a buffered aqueous solution containing a blocking agent according to the disclosure is given in the appended examples. The blocking takes preferably place at room temperature for usually 1 to 4 hours, preferably 1 hour.

Alternatively to step (b'), the method also being described herein may comprise a step (b") to be carried out subsequent to step (b) and previous to step (c):

(b") blocking unbound binding sites using an aqueous solution containing 0.5-10 % (w/w) substances selected from the group consisting of (poly)peptides (e.g. albumin, gelatin or collagen), hydroxyethylstarch (HES), mannitol, sorbitol and polyethyleneglycol (PEG), milk, soya, wheat or egg derived protein.

In an even more preferred example step (b') or (b") may further comprise performing one or more washing steps using an aqueous solution as defined as suitable for step (b) above after blocking. An aqueous solution suitable in this embodiment is e.g. PBS with 1% human albumin. This one or more washing steps are effected to remove excess blocking agent. The washings are usually effected for 10 seconds to 10 minutes, depending on the surface of the carrier, preferably at room temperature.

In a preferred embodiment the carrier in step (c) is incubated in an aqueous solution comprising one or more substances selected from the group consisting of albumin, hydroxyethylstarch (HES), and chaperones. Chaperones are commonly known in the art as proteins assisting in the folding process, transport or translocation of proteins or exhibiting protective properties when in contact with other proteins. They are found in prokaryotes as well as in eukaryotes. Prominent examples of chaperones are proteins belonging to the family of heat shock proteins, such as Hsp 60 and Hsp 10, Hsp 70, Hsp 90 and Hsp 100. Other chaperones are the bacterial GroES/GroEL complex or the mammalian DnaK protein.

Chaperones also known as compatible solutes are also present in extreme halophilic bacteria. They are amphoteric, water-binding organic molecules that form large hydration shells and reported to stabilize proteins and nucleic acids in extremophiles in their native state. Compatible solutes are characterized by their property to be excluded from protein surfaces. This property enables cells to accumulate high concentrations of these compounds without affecting structure and function of their proteins at the same time. Furthermore, the resulting non-uniform distribution of compatible solutes within the cell has stabilizing effects on the structure of proteins. Until now, synthetic derivatives of these chaperones have been designed. Natural compatible solutes as well as synthetic derivatives thereof are also envisaged in the present invention. Exemplary compatible solutes are ectoin ((4S)-2-methyl-3,4,5,6-tetrahydropyrimidine-4-carboxylic acid), hydroxyectoine, betaine, glycine-betaine, proline-betaine and derivatives thereof.

Alternatively, the carrier in step (c) is incubated in accordance with a preferred embodiment in an ionic liquid. An ionic liquid is a salt in which the ions are poorly coordinated, which results in these solvents being liquid. At least one ion has a delocalized charge and one component is organic, which prevents the formation of a stable crystal lattice. Properties, such as melting point, viscosity, and solubility of starting materials and other solvents, are determined by the substituents on the organic component and by the counterion.

Some ionic liquids, such as ethylammonium nitrate, are in a dynamic equilibrium where at any time more than 99.99% of the liquid is made up of ionic rather than molecular species. Whereas in the broad sense, the term includes all molten salts, for instance, sodium chloride at temperatures higher than 800 °C, in context with the present invention, the term "ionic liquid" defines liquid salts whose melting point is relatively low (below 100 °C). In particular, the salts that are liquid at room temperature are called room-temperature ionic liquids, or RTILs which are especially preferred in the present invention. RTILs consist of bulky and asymmetric organic cations such as methylimidazolium (mim), 1-alkyl-3-methylimidazolium (e.g. ethyl-mim (emim), butyl-mim), pyridinium (py), 1-alkylpyridinium (e.g. (butyl-py, butal-methyl-py), N-methyl-N-alkylpyrrolidinium or ammonium ions. A wide range of anions is employed, from simple halides, which generally inflect high melting points, to inorganic anions such as tetrafluoroborate and hexafluorophosphate ($PF_6$) and to large organic anions like bis-trifluorsulfonimide, triflate or tosylate. Ionic liquids with simple non-halogenated organic anions such as formate, alkylsulfate, alkylphosphate or glycolate may also be used. As an example, the melting point of 1-butyl-3-methylimidazolium tetrafluoroborate or [bmim][$BF_4$] with an imidazole skeleton is about -80 °C, and it is a colorless liquid with high viscosity at room temperature. One of the first RTILs was a mixture of [emim]Cl with $AlCl_3$ forming a series of equilibria between [emim][$AlCl_4$], [emim][$Al_2Cl_7$], and [emim][$Al_3Cl_{10}$]. This RTIL is not water stable. An exemplary water-insoluble RTIL is [bmim][$PF_6$]. Exemplary ionic liquids are (1-n-Butyl-3-methylimidazolium-hexafluorophosphat (BMIM[PF6]), BMIMbis(trifluormethansulfon)-imide (BMIM[Tf2N]), Methyltrioctylammonium-[Tf2N] (OMA[Tf2N]) or 1,3-di-methyl-imidazolmethylsulfate [MMIM] [MeSO4].

Ionic liquids are suggested to be suitable to protect substances coated thereby from potentially harmful material influences or radiation. In the context of the present invention, ionic liquids are believed to protect the biological material from decomposition upon sterilization.

In line with the invention it is further preferred that the material of the carrier produced by the method of the invention is of porous structure.

To achieve a compact design of the coated carrier to be used as a (medical) product it is preferred that the carrier displays a relatively large surface as compared to its overall measurement. This can be achieved e.g. by using a foam with an open porous structure, a fleece (wadding) or a setting using many parallel small tubes or filaments, for example those similar to a porous hollow fiber design currently used in hemodialysis (see e.g. http://www.fmc-ag.com/internet/fmc/fmcag/agintpub.nsf/ Content/Modern_hemodialysis_+the_first_hollow-fiber_dialyzers_2004). The design preferably allows for a free flow of whole blood or the above described other body fluids, and the carrier is preferably rheologically optimized by a minimum pressure difference between the inflow and outflow and a blood flow of <1m/sec at any point within the device. It is preferably without "dead ends" and optimized for contacts of blood constituents with the active surface of the matrix. The specific advantage of a carrier with porous structure is the increase of the carrier surface to which the biological material can be attached to. Namely, the amount of biological material which can be attached/embedded on a carrier is increased by the increase of the carrier surface per carrier volume.

More preferably, the material of the carrier is characterized by a surface/gaseous volume ratio in a range of 30 $cm^{-1}$ and 300 $cm^{-1}$. The surface of a carrier is understood as the sum of the surfaces of all trabeculae. The gaseous volume is understood as the sum of the volume of gas in all trabeculae of a carrier with porous structure.

The surface/gaseous volume ratio may be e.g. determined by cutting a small piece of foam with a defined overall measurement. All trabeculae of said piece of foam are counted as well as measured microscopically. Using the mean length and diameter of a trabecula as well as the count of the trabeculae per $cm^3$ the specific surface (xx $cm^2/cm^3$, which is $cm^{-1}$ and describes the inner surface of a defined volume of material) can be obtained mathematically (assuming the trabeculae are round in shape).

The material of the carrier with porous structure is preferably characterized by a material volume ratio uncompressed/compressed in a range of 4 to 40.

The term "material volume ratio" is understood in the context of the invention as a ratio between uncompressed and compressed porous material, comprising solid and gaseous components.

In the case of a porous, elastic foam like e.g. PU-foam, the ratio can for example be determined principally as follows. A cylindrical piece of foam with a volume of 20 ml (uncompressed) is placed into a syringe with a volume of 20 ml, and the compressed volume is readable on the marks of the syringe after pressing the plunger fully down. The force that is used for the compression of the material (in the case of PU porous foam) is defined as at least 20 $kg/cm^2$. The endpoint of volume reduction is defined as the volume at which a doubling of the compression pressure (in the case of PU porous foam the pressure will be 40 $kg/cm^2$, respectively) will not result in a further volume reduction of up to 10 %. This procedure (doubling of pressure force) may be repeated until the material does not undergo further volume reduction of up to 10%.

In line with the method of the invention it is further preferred that the material of the carrier is selected from the group consisting of glass, polyurethane (PU), polyester, polysulfone, polyethylene, polypropylene, polyacryl, polyacrylnitril, polyamid, PMMA, fleece wadding, open porous foam plastic or glass and reticular plastic or glass and structures derived from marine sponges (porifera). For example, a polyester fleece may be used as described for the device LG6 from Pall, Dreieich, Germany. A nonlimiting example for glass-filaments includes biofilter membranes for a blood transfusion set with Leukocyte Removal Filter as distributed by Nanjing Shuangwei Science & Technology Industries Co. LTD. Examples for suitable marine sponges are described e.g. in D. Green et al., Tissue Engineering. (2003) Vol. 9, No. 6: 1159-1166. The material that may be used in the method of the present invention may have the structure of hollow fiber. A hollow fiber package is preferably characterized by a material volume ratio uncompressed/compressed in a range of 1 to 10 and/or the surface/gaseous volume ratio is in a range of 200 $cm^{-1}$ and 2000 $cm^{-1}$.

In a further preferred embodiment of the method of the invention the solution in step (a) is an aqueous solution. In an alternatively preferred embodiment of the method of the invention the solution in step (a) is a non-aqueous solution. Examples for corresponding solutions and for preferred solutions have been described herein above.

The antibodies described in the context of the disclosure are capable to specifically bind/interact with an epitope. The epitope may be a polypeptide structure as well as compounds which do not comprise amino acids. The term "specifically binding/interacting with" as used in accordance with the present disclosure means that the antibody or antibody fragment does not or essentially does not cross-react with an epitope of similar structure. Cross-reactivity of a panel of antibodies under investigation may be tested, for example, by assessing binding of said panel of antibodies under conventional conditions to the epitope of interest as well as to a number of more or less (structurally and/or functionally) closely related epitopes. Only those antibodies that bind to the epitope of interest in its relevant context (e.g. a specific motif in the structure of a protein) but do not or do not essentially bind to any of the other epitope are considered specific for the epitope of interest. Corresponding methods are described e.g. in Harlow and Lane, Antibodies: A Laboratory Manual, Cold Spring Harbor Laboratory Press, 1988 or Harlow and Lane, Using Antibodies: A Laboratory Manual, Cold Spring Harbor Laboratory Press, 1999)

The antibody or antibody fragment or antibody derivative specifically binds to/interacts with conformational or continuous epitopes of an antigen. A conformational or discontinuous epitope is characterized for polypeptide antigens by the presence of two or more discrete amino acid residues which are separated in the primary sequence, but come together on the surface of the molecule when the polypeptide folds into the native protein/antigen (Sela, (1969) Science 166, 1365 and Laver, (1990) Cell 61, 553-6). The two or more discrete amino acid residues contributing to the epitope are present on separate sections of one or more (poly)peptide chain(s). These residues come together on the surface of the molecule when the (poly)peptide chain(s) fold(s) into a three-dimensional structure to constitute the epitope. In contrast, a linear or continuous epitope consist of two or more discrete amino acid residues which are present in a single linear segment of a (poly)peptide chain.

The antibody may be a monoclonal or a polyclonal antibody of any class of antibody.

The term "antibody" also comprises derivatives or fragments thereof which still retain the binding specificity. The antibody of the invention also includes embodiments such as synthetic, chimeric, single chain and humanized antibodies, as well as antibody fragments.

The term "antibody fragment" relates to fragments, such as Fab, $F(ab_2)'$ or Fv fragments. The term "antibody derivative" defines in the context of the invention chemically modified antibodies and antibody fragments. This includes scFv fragments, single domain antibodies etc. Accordingly, antibody derivatives are often (poly)peptides derived from antibody molecules and/or (poly)peptides which are modified by chemical/biochemical or molecular biological methods. The minimal requirement for the specific interaction of an antibody fragment with its specific antigen is the presence of one or more CDRs from the variable heavy chain ($V_H$) and the variable light variable chain ($V_L$) of the parent antibody in a context which allows for the fitting of the antibody fragment and the epitope. Such a context can be provided by the use of a suitable framework of an antibody. As known in the art the term "framework" defines in the context of an antibody or antibody derivative the amino acid sequence which functions as a spacer between the CDRs as well as extends N-terminally and C-terminally thereof and provides for a structure which allows the formation of the antigen binding site by the CDRs. A modification of the framework or CDR sequences, e.g. to improve the binding affinity by molecular biological methods may comprise modification of the (poly)peptides using conventional techniques known in the art, for example, by using amino acid deletion(s), insertion(s), substitution(s), addition(s), and/or recombination(s) and/or any other modification(s) (e.g. posttranslational and chemical modifications, such as glycosylation and phosphorylation) known in the art either alone or in combination. Methods for introducing such modifications in the DNA sequence underlying the amino acid sequence of an immunoglobulin chain are well known to the person skilled in the art; see, e.g., Sambrook et al., Molecular Cloning: A Laboratory Manual, Cold Spring Harbor Laboratory Press, 2nd edition 1989 and 3rd edition 2001; Gerhardt et al., Methods for General and Molecular Bacteriology, ASM Press, 1994; Lefkovits, Immunology Methods Manual: The Comprehensive Sourcebook of Techniques, Academic Press, 1997; or Golemis, Protein-Protein Interactions: A Molecular Cloning Manual, Cold Spring Harbor Laboratory Press, 2002.

As noted herein above, it is further preferred that the antibody is a monoclonal antibody.

It is most preferred that the (monoclonal) antibody is of the IgG, IgM or IgY class.

In line with a further preferred embodiment of the method of the invention the biological material attached in step (b) is covalently bound to said carrier. Methods, how to achieve an attachment of the biological material to the carrier via a covalent bond have been described herein above.

In line with a further preferred embodiment of the method of the invention the steps (a) to (c) are effected in a system of rotating tubes that contain the carrier.

Moreover, in line with a further preferred embodiment of the method of the invention the solutions are rotated in the system of tubes via a pump.

In a different particularly preferred embodiment of the method of the invention the carrier made of a material having a porous structure comprises at least one further material. For example, in case the carrier of the invention is made of one of the materials as defined above providing one or more chemical entities, on or more other materials can be added to provide different chemical entities or to alter the properties of the carrier. The same holds true in case the carrier is made of two, three or more materials. In other terms, besides the substances defined above, the carrier used for coating according to the present invention comprises at least one further material. The material can be worked into the carrier upon its production by mixing the ingredients or by filling the carrier with the one or more additional materials. Preferably, said at least one further material is selected from the group consisting of carbon, $SiO_2$, hydroxyethylstarch (HES) and biotin. Examples for a porous carrier comprising one further material include e.g. carbonfilled polyurethane and polyether foam from Kinetic Concepts Inc. (KCI, Texas, USA).

In line with a further preferred embodiment of the method of the invention the solution in steps (a) to (c) is an aqueous solution which comprises 0.5 to 10 % albumin (v/v) and 0.5 to 5 % mannitol (v/v). It is particularly preferred in accordance with the invention that the aqueous solution in steps (a) to (c) comprises 0.6 to 3 % albumin (v/v). The aqueous solution preferably comprises 0.6 to 3 % of mannitol (v/v).

It is also described herein that the solution in step (a) may be an alcohol with silane 0.1-10 % (v/v), the one in step (b) may be an aqueous solution which comprises the biological material (for example antibodies) and (c) may be an aqueous solution which comprises 0.5 to 10 % albumin (v/v) and 0.5 to 5 % mannitol (v/v). It is particularly preferred in accordance with the disclosure that the aqueous solution in steps (a) to (c) comprises 0.6 to 3 % albumin (v/v). The aqueous solution preferably comprises 0.6 to 3 % of a sugar (e.g. mannitol) (v/v).

The method of the invention further comprises a step (e) of sterilizing the solid coated carrier. The ability to sterilize the solid coated carrier produced by the method of the invention allows, *inter alia,* the production of the solid coated carriers under non-sterile or semi-sterile conditions, wherein the so produced carriers may still be used in the method of the invention which involves the contact of the solid coated carrier with a body liquid *in vivo* or *ex vivo.* This represents a further superior feature of the method and the so produced carrier of the invention since, due to that feature, the costs for the production of the solid carrier can be reduced compared to costs for the production of carriers which may not be sterilized. This is because, in the production process, sterile conditions are not required. As it is well known, conventionally prepared carriers are not suitable for a sterilization and must be produced under completely sterile conditions. Moreover, a cost contributing feature is that said carriers may be recycled after their use by sterilization. The recycled carriers may subsequently be used in a further treatment of the same or a different patient or in a further or the same method for diagnosing which again involves the contact of the carrier with a body liquid *in vivo* or *ex vivo.*

Preferably, the sterilization or recycling of the carrier is effected by ethyleneoxid (EO), beta radiation, gamma radiation, X-ray, heat inactivation, autoclaving or plasma sterilization depending on the carrier material. It is most preferred that the carrier is sterilized by β- or γ-radiation. Suitable in this embodiment is β-radiation with a dose of 25 kgray using an electron accelerator with 10MeV. To a certain extent sterilization with ethyleneoxide can be applied to the carrier of the invention. In general, the method of sterilization has to be chosen in order not to harm the desired activity of the coated biological material. This can be effected with fragments of cells, (poly)peptides, especially antibodies or receptors, polynucleotides or polysaccharides or complexes thereof as defined above. For cells of any kind, suitable means of sterilization are not known to date. Thus, embodiments where living cells are used as the biological material and the carrier is sterilized are not part of this invention.

The method of the invention may further comprise a step (f) of washing the coated carrier after step (d).

The washing step referred to above serves to partially or completely remove the coating layer applied in step (c), i.e. the second coating layer. The washing step can be applied regardless of whether the coating layer completely or partially covers the biological material. In this way, the biological material, preferably the functional parts of the biological material such as e.g. antigen binding sites of antibodies, catalytic sites of enzymes or protein interaction sites on (poly)peptides in general are again made partially or fully accessible on the surface of the carrier of the invention and can fulfill their function. The extent of uncovering the biological material from the second covering layer can be quantitatively determined using tests based on the biological function of the biological material used. In the case of antibodies used as biological material, the accessibility of the functional parts, i.e. in this case the antigen binding sites can e.g. be determined using the specific antigen coupled to a detectable marker as is well known to the person skilled in the art. Similarly, the accessibility of the catalytic site of an enzyme or the protein interaction site of a (poly)peptide in general can be assessed by contacting a substrate of the respective enzyme or a labeled interaction partner or interacting fragment thereof with the carrier and detecting the turn-over of the substrate or the presence of the interacting partner. To ensure that the carriers of the present invention applied for the purposes as described elsewhere in this application are still sterile after the washing step and the determination of accessibility of the functional part of the biological material, the testing is done on one carrier whereas a different carrier from the same batch is applied for its purpose.

The washing step is preferably carried out using a solution adapted to the components comprised in the second coating layer and which can comprise adequate salts and/or enzymes. Enzymes added may serve to decompose certain components of the second layer such as e.g. proteins or starch derivatives. Even with an enzyme contained in the washing solution, the step (f) is still regarded as a washing step to remove parts or all of the second coating layer. The temperature and pH value of the washing solution are equally adapted to the specific requirements arising from the component(s) of the second coating layer used. The simplest washing solution could e.g. be an isotonic salt solution. For certain applications, the solution can also comprise plasma surrogates. Depending on the application of the coated carrier, the washing step can also be effected with body fluids, e.g. ex vivo by exposing the carrier to the blood or plasma stream in the course of e.g. dialysis, heart-lung-machines or compresses for wounds.

Apart from the exposure of parts of the biological material or the complete surface of the biological material, the above washing step is useful to remove particles loosely attached to the carrier to obtain a carrier with all parts tightly attached.

The washing step is carried out after the sterilization process but preferably immediately prior to use. This is to ensure that the freshly sterilized coated carrier of the invention is not contaminated by the early contact of the carrier with a (washing)solution.

Also described herein is a solid coated carrier producible or produced by the method of the disclosure.

Also described herein is a solid coated carrier to which biological material is attached, wherein the biological material is embedded into a coating matrix consisting of a first layer of at least one silane which is in direct contact with the carrier and a second layer partially or completely covering the first layer consisting of at least one substance selected from the group consisting of at least one (poly)peptide, at least one amino acid, starch, at least one sugar, at least one phosphate, at least one polyalcohol and polyethyleneglycol (PEG) or a mixture thereof. More preferred, the at least one substance of the second layer is selected from the group comprising inter alia human or animal serum and proteins of such sera, (e.g. albumin), chaperones, milk, egg proteins, plant derived proteins (e.g. soya, wheat) including hydrolysates of said proteins (e.g. gelatin, collagen), the group of amino acids (preferably glycine, aspartic and/or glutamic acids, proline, arginine, alanine, asparagine, aspartic acid, glutamic acid, glutamine, carnitine, ornithine, hydroxyproline, cysteine, homocysteine, citrulline, inuline, phenylanine, lysine, leucine, isoleucine, histidine, methionine, serine, valine, tyrosine, threonine, tryptophan) or derivates of amino acids (e.g. n-acetyl-tryptophan, beta-alanine, melanin, DOPA), the group of sugars (preferably glucose, saccharose, sucrose), poly alcohols (preferably sorbitol, mannitol, glycerol, xylitol), polyethyleneglycol (PEG), hydroxyethylstarch (HES), the group of phosphates (e.g. sodiummonodihydrogenphosphate, disodiumhydrogenphosphate) or a mixture thereof. Alternatively, the second layer consists of an ionic liquid or a compatible solute.

It is preferred that the solid coated carrier is produced in accordance with the method of the disclosure.

Definitions and explanations *inter alia* of the carrier, the biological material, the attachment of the biological material and the material of the coating matrix provided in accordance with the method of the disclosure *mutatis mutandis* apply to the solid coated carrier of the disclosure.

Also, the particular superior features of the solid coated carrier of the present disclosure have been described herein above in the context of the characterization of the method of the disclosure.

As also described in the context of the method of producing a solid coated carrier of the disclosure it is also preferred that the material of the carrier is selected from the group consisting of glass, polyurethane, polyester, polysulfone, polyethylene, polypropylene, polyacryl, polyacrylnitril, polyamid, PMMA, fleece wadding, open porous foam plastic, reticular plastic and structures derived from marine sponges (porifera).

According to a preferred example of the solid coated carrier of the disclosure the biological material is selected from the group consisting of eukaryotic cells, fragments of eukaryotic cells, prokaryotes, fragments of prokaryotes, archaebacteria, fragments of archaebacteria, viruses and viral fragments. Preferably, the fragments of eukaryotic cells, fragments of prokaryotes, fragments of archaebacteria, or viral fragments are selected from the group consisting of (poly)peptides, oligonucleotides, polynucleotides and polysaccharides.

In an alternative example of the solid coated carrier of the disclosure the biological material is selected from the group consisting of (poly)peptides, oligonucleotides, polynucleotides and polysaccharides which are produced synthetically or semisynthetically or recombinantly.

In an also preferred example of the carrier of the disclosure the at least one (poly)peptide of the second layer is albumin, the starch of the second layer is hydroxyethylstarch (HES) and/or the at least one sugar of the second layer is mannitol or sorbitol.

It is preferred that the material of a carrier having a porous structure is characterized by an surface/gaseous volume ratio in a range of 30 $cm^{-1}$ and 300 $cm^{-1}$.

It is also preferred that the material of the carrier having a porous structure is characterized by a material volume ratio uncompressed/compressed in a range of 4 to 40.

In a further preferred disclosure the material of the carrier is hollow fiber. A hollow fiber package is preferably characterized by a material volume ratio uncompressed/compressed in a range of 1 to 10 and/or the surface/gaseous volume ratio is in a range of 200 $cm^{-1}$ and 2000 $cm^{-1}$.

It is additionally preferred that the at least one silane of the first layer is selected from the group consisting of alkoxysilanes, organofunctional silanes, hydrogensil(ox)anes, siloxanes, organosilanes comprising silyl compounds with other functional groups. Examples of appropriate silanes have been provided above.

In line with the disclosure the second layer of the carrier may be a preferably dried mixture comprising albumin and mannitol. Preferably, said mixture further comprises polyethyleneglycol (PEG). In particular, in accordance with the present invention, the mixture is applied to the carrier in liquid form and subsequently dried on the surface of the carrier of the invention. Drying is preferably effected by air-drying. In another preferred embodiment, the second layer comprises chaperones or consists of an ionic liquid or a compatible solute as has been described above in connection with the method of the present invention.

In a further preferred example of the disclosure the biological material is covalently bound to said carrier. Methods to achieve a covalent binding of biological material to a solid carrier are described herein above and in the appended example 2.

It is further preferred for the solid coated carrier of the disclosure that the (poly)peptide in item (b), which is embedded in the coating matrix, is a receptor. More preferably, said receptor is an antibody or an antibody fragment. It is particularly envisaged that the antibody may be a monoclonal antibody.

The (monoclonal) antibody embedded into the coating matrix of the carrier of the disclosure may be an antibody of any class of antibody. Preferably, the antibody is of the IgG, IgY or IgM class.

The (poly)peptide may also be a cytokine or an angiogenesis-promoting growth factor as has been described above in connection with the method of the present disclosure. It is more preferred that the cytokine is a BMP, most preferably BMP-2 or BMP-7. Also, the angiogenesis-promoting growth factor may be PDGF.

It is particularly preferred that the carrier of the disclosure comprises at least one further material. For example, in case the carrier of the invention is made of one of the materials as defined above providing one or more chemical entities, on or more other materials can be added to provide different chemical entities or to alter the properties of the carrier. The same holds true in case the carrier is made of two, three or more materials. In other terms, besides the substances defined above, the carrier used for coating according to the present disclosure comprises at least one further material. The material can be worked into the carrier upon its production by mixing the ingredients or by filling the carrier with the one or more additional materials. Preferably, said at least one further material is selected from the group consisting of carbon, $SiO_2$, HES and biotin.

In another preferred example of the present disclosure the carrier is sterilized by β- or γ-radiation. Suitable in this example is β-radiation with a dose of 25 kgray using an electron accelerator with 10MeV.

In an alternative embodiment the present invention provides the use of a solid coated carrier produced or producible by the method of the invention for the preparation of a medical device for the contacting, filtering or cleaning of blood, lymph or liquor cerebrospinal of a patient.

The term "(medical) device" defines in the context of the disclosure a device which comprises a solid coated carrier produced by a method of the invention. Examples of such devices are exemplified herein below. The device is suitable to enable a contacting, filtering or cleaning of blood, lymph or liquor cerebrospinal of a patient e.g. by connecting the device with the circulation of the body fluid of the patient and thereby treating a patient as described herein above (in the context of the method for the production of the solid coated carrier). Moreover, the (medical) device may be suitable for the qualitative or quantitative detection of compounds as well as trapping certain cells, e. g. stem cells, in a sample of a body fluid of a patient as also described in part herein above.

Stem cells comprise pluripotent, multipotent or totipotent stem cells. By trapping certain cells or one or more cell populations with the carrier of the disclosure suitably coated with one or more biological materials as defined above which are specifically binding to these cells or cell populations(s) by contacting in a sample of a body fluid, at least a part of these cells or cell population(s) can be removed from the sample. In general, different cell lines, cell species or developmental stages of cells can be distinguished by the presence of different antigens on the cell surface. This enables for selective trapping of the desired cells by coating the carrier of the disclosure with e.g. a corresponding receptor for the antigen or an antibody directed against the antigen.

The term "contacting, filtering or cleaning" as used throughout the present application refers to alternative means of removing one or more compounds from a solution. Accordingly, the term "contacting" may be an initial step of filtering and cleaning.

The disclosed preferably medical device is useful in therapies which comprise the detoxification of body fluids from toxins such as dioxin, botulism toxin, tetanus toxin, LPS, septic poisons etc. Moreover, it is useful in the treatment of bacterial or viral diseases, especially of diseases with a major virus load in the blood or other body fluids (e.g. haemorrhagic fevers, hepatitis a, b, c, d, e, HIV, dengue fever) or a major bacterial load in the blood or alternative body fluids (e.g. sepsis with meningo- or pneumococcal bacteria). A further envisaged alternative application of the disclosed medical device is the use in a therapy which requires the stimulation or elimination of certain cells of a patient. An elimination of a specific population of cells is e.g. indicated in the treatment of proliferative diseases (e.g. minimal residual cancer), autoimmune diseases or a treatment of diseases caused by organ transplantation. A stimulation of a specific population of cells is e.g. indicated in the treatment of a disease which is cured or alleviated by the activation of cells immune system. The population of activated cells is suitable to eliminate a specific population of diseased cells.

Further, the trapping of stem cells using appropriate antibodies affixed to the solid carrier (for example anti-CD34 or CD133 is a useful application of the device. After trapping, the cells are harvested by enzymatic release, cold fluids, mechanical force (vibration etc.) or a combination of these techniques.

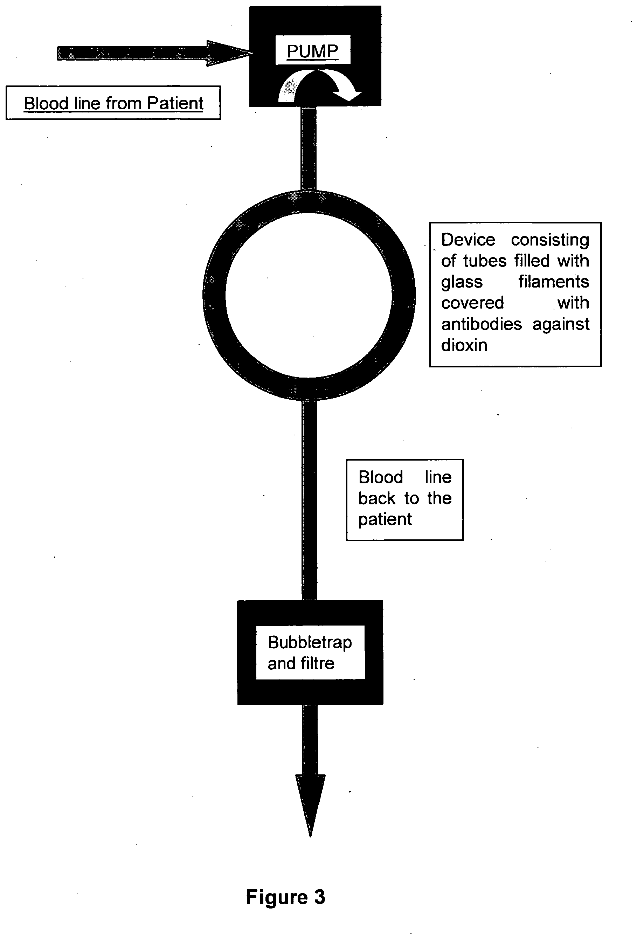

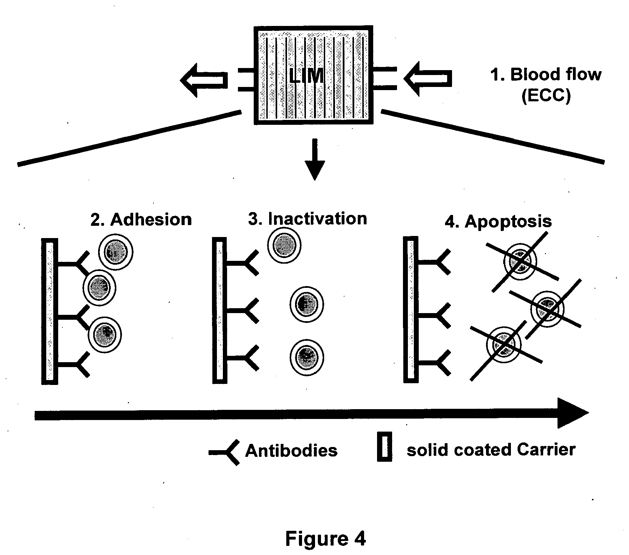

Examples for embodiments of the disclosed (medical) devices are schematically depicted in figures 3 and 4.

One embodiment of the medical device includes transient or permanent implants characterized by a surface which itself is a solid coated carrier or to which solid coated carriers produced according to the method of the invention are attached to. This includes osteosynthesis devices, materials used for reconstructive surgery etc. as well as a novel class of stents. Such novel class of stents may have immune catalyzing features (stimulatory, inhibitory or eliminating features as described above).

Further embodiments of the medical device include catheters, circuits, devices that are incorporated into the extracorporeal systems, e.g. arterial filters, oxygenators, reservoirs, the Leukocyte Inhibition Module (LIM), Leukocyte Stimulation Module (LSM), Tolerance Induction Module or artificial lymph nodes which comprise solid coated carriers or to which solid coated carriers produced according to the method of the invention are attached to. Such modules have been described e.g. by Scholz M et al. ASAIO J 2005;51:144-7; Scholz M, Cinatl J Med Res Rev 2005;25:331-42; Scholz M et al. J Thorac Cardiovasc Surg 2004; 127:1735-42; Moreno JB et al. Perfusion 2004;19:11-6.

Moreover, further alternative embodiments of the medical device are devices which interior walls are solid coated carriers or to which solid coated carriers are attached to including ex vivo vaccination container, apheresis devices, stem cell isolation devices, purging devices, syringes and devices for transient or permanent blood storage (e.g. in blood banks, but also laboratory equipment in routine or research laboratories). An example for a diagnostic medical device is an ELISA plate wherein the surface of the plate (or at least of the reaction wells) is a solid coated carrier or solid coated carriers are attached to.

Examples for the above indicated diseases comprise severe hyperlipidemia of variuos origin, homozygous familial hypercholesterolemia, heterozygous familial hypercholesterolemia, defective Apo B-100, isolated Lipoprotein (a) elevation, systemic lupus erythematosus (SLE), Sjögren's syndrome, mixed connective tissue disease, dilated cardiomyopathy (DCM), diseases associated with inhibitors to coagulation factors, idiopathic thrombocytopenic purpura (ITP), thrombotic thrombocytopenic purpura (TTP), autoimmune hemolytic anemia, hyperviscosity syndrome in hypergammaglobulinemia, myasthenia gravis, Guillain-Barre syndrome, chronic inflammatory demyelinating polyneuropathy (CIDP), dysproteinemic polyneuropathies, bone marrow transplantation, endocrine orbitopathy, diabetes mellitus type I (IDDM), Goodpasture's syndrome, nephropathies due to immunoglobulin or immune complex deposits, cryoglobulinemia, pemphigus, atopic dermatitis, graft-versus-host (GvH) diseases, host-versus-graft (HvG) diseases, and various forms of vasculitis.

The invention also provides a method for the manipulation of the composition of blood, lymph or liquor cerebrospinal of a patient comprising the contacting of the blood, lymph or liquor cerebrospinal with a solid coated carrier produced or producible by the method of the invention.

The term "manipulation of the composition" of a body liquid defines a process of affecting the characteristic features of the body liquid or a sample of a different body liquid. This comprises a filtration or cleaning of the liquid. In such a case, biological material, e.g. (a) receptor(s), on the carrier will bind to component(s) of the body fluid and retain said component(s). This step is preferably effected under physiological conditions as defined above. Also comprised by this definition is the induction of a change of the activation status of cells comprised in the sample as well as the elimination of one or more specific populations of cells.

The manipulation of the composition of blood, lymph or liquor cerebrospinal of a patient may be effected in accordance with the invention in vivo, ex vivo or in vitro.

In a subsequent step of the ex vivo or in vitro embodiment of the method for the manipulation of the composition of blood, lymph or liquor cerebrospinal of a patient, the blood, lymph or the liquor is removed from the carrier after the contacting with the solid coated carrier.

It is also described herein that the contacting of the carrier comprising the embedded biological material in the example where the biological material is a cell, e.g. donor or genetically engineered/recombinant cell, enables the secretion of substances produced by the embedded cell into the body fluid of a patient. The secreted substance is the effective substance in the treatment of a disease of a patient. Diseases that can treated by such an approach comprise e.g. diabetes mellitus (use of islet cells or other cells secreting insulin), endocrinal diseases (e.g. use of cells from the thyroid gland or the pineal body or other cells secreting a hormone helpful in the treatment of a disease). Thus, the method for the manipulation of the composition of blood, lymph or liquor cerebrospinal of a patient described herein enables a treatment of a patient with a minimized risk of a side effect. This is because the donor or genetically engineered/recombinant cells if applied in an in vitro/ex vivo scenario, will not enter the patient. Even if applied in vivo, these cells will not be released into the blood stream or other body fluids due to the described mode of attachment to the solid carrier.

Preferably, the above described use and the method of the invention are characterized by a manipulation of the composition of blood, lymph or liquor cerebrospinal, which is effected in a batch container which contains the solid coated carrier. Alternatively, the manipulation of the composition of blood, lymph or liquor cerebrospinal is effected in a flow through container which contains the solid coated carrier.

In a further embodiment the invention provides a method for the diagnosis of a disease. The method comprising the steps of:

(a) contacting body liquid obtained from a patient with a solid coated carrier produced or producible by the method of the invention into which an antibody is embedded under suitable conditions for a specific binding of a pathogen or marker protein indicative for the disease to the embedded antibody; and

(b) detecting whether the pathogen or marker protein indicative for the disease has been bound to the embedded antibody.

The described method may be carried out in vivo, ex vivo or in vitro.

Examples for diseases to be diagnosed with the diagnostic method of the invention comprise the diseases described herein above in the context of the medical use of the invention. The recited pathogen or marker protein may e.g. be detected by the use of antibodies like anti-p24 (HIV) (see e.g. Schupbach et al. J Aquir Immune Defic Syndr 2005;40:250-6.) or anti-gB (HCMV) (see e.g. Just-Nubling G et al. Infection 2003;31:318-23).

Suitable conditions for a specific binding of a pathogen or marker protein indicative of the disease to the attached antibody may be achieved by contacting a sample of the body liquid of the patient with antibody covered surfaces according to the disclosure under physiological conditions.

The diagnostic method of the invention may comprise the step of incubating the material of a sample of the body liquid under cell culture conditions in order to enrich a pathogen such as a virus, bacterium or a single cell eukaryotic pathogen.

Moreover, the invention provides a use of diagnostic composition comprising a solid coated carrier according to the invention. The diagnostic composition of the invention will preferably be used for the diagnosis of a disease.

Examples for diseases to be diagnosed with the diagnostic composition of the invention have been described herein above.

In addition, the present invention provides for a method of purifying a compound comprising contacting a mixture comprising the compound to be purified with the solid coated carrier produced or producible by the method of the invention. Depending on the molecule attached or coated to the carrier and specifically binding to a compound, the method of the present invention can be applied to purify e.g. proteins, nucleic acids, protein complexes or other molecules of biological or inorganic origin.

The method of the invention is also suitable to separate proteins of the same kind having different phosphorylation states or having different post-translational modifications using the coated carrier of the invention, wherein the molecule coated to the carrier specifically recognizes one of these states or modifications.

The principle of the method of the present inventions corresponds to that of affinity purification which is commonly known in the art. The person skilled in the art appreciates that different formats can be used to carry out the method of the present invention. For example, commonly applied formats are columns packed with the affinity material, i.e. the coated carrier produced or producible by the method of the invention.

In accordance with the present invention, the molecule attached to the carrier is an antibody as e.g. described above.

The figures show:

**Figure 1:**

Scheme of the coating procedure for the immobilization of biological material. Exemplified is the procedure for biological material such as antibodies or cells (or fragments) and their functional maintenance under stress conditions. The figure depicts a preferred method also comprising a sterilization and an additional washing step.

**Figure 2:**

Flow cytometric dot blot analyses which depicts the experimental result described in the appended example 1. The analysis reveals the maintenance of antibody-mediated apoptosis induction after EO-sterilization.

**Figure 3:**

Schematic design of an example for a therapeutic toxin trap (e.g. a dioxin trap). The exemplified design allows a low blood flow and priming volume. Thus, it may also be used in diverse catheter systems such as Sheldon catheters, e.g. after/during trauma, shock and sepsis and other settings in critical care.

**Figure 4:**

Schematic depiction of an example for a (medical) device consisting of a plastic housing carrying a polyurethane foam with immobilized antibodies (e.g. against CD95). Neutrophils can transiently adhere to the antibodies embedded by the coating. The binding of the antibody can trigger a signal for the inactivation of the neutrophils.

The examples illustrate the invention.

**Example 1 (for comparison only):**

**Induction of apoptosis in a cell population using the solid coated carrier**

The goal of the experimental approach was to determine the protective effect of the coating procedure in terms of functional activity of immobilized anti-Fas (CD95; APO-1) IgM-antibodies after sterilization with ethyleneoxid or beta-radiation.

**Coating of NUNC 8-Well chamber slides and immobilization of anti-Fas IgM :**

Two 8-well chamber slides were used for coating. Each well was treated with 250 µl methanol with 4% (2-5%) N-[3-(Trimethoxysilyl)propyl]ethylenediamine for 30 min at room temperature. After inverse centrifugation and 10 min drying under laminar flow, part of the wells were incubated with 200 µl antibody containing buffer

(1:100 represents 1μg antibody per well on 0,64 cm²) for 1h at 37° C and 90 % humidity. Untreated wells served as controls. Subsequently, wells were treated with a blocking solution (PBS with 5% serum and 5% mannitol) for 30 min., washed three times with the blocking solution and dried as described above. The coated wells can be stored at 4° C for several weeks. Sterilization was carried out at 1,7 bar, 45° C, 180 min 6% EO, 94 % CO2, with a maximum temperature of 47°C.

**Assay with Jurkat cells:**

Three to five days after splitting, 1x10⁵ Jurkat cells were added to each of the the prepared wells (according to the description above) in the presence of 2 % serum and incubated overnight. After 48h, 8μl propidium iodide (PI) and 1μl annexin V were added to each well. Apoptotic cells bind annexin V and necrotic cells take up PI. After 15 min cells were gently removed from the wells and the fluorescence determined by flow cytometry (apoptosis: FL1; necrosis: FL2).

Wells were washed carefully with PBS and stored at 4° C to test the shelf life of the coating procedure at later time points.

**Results:**

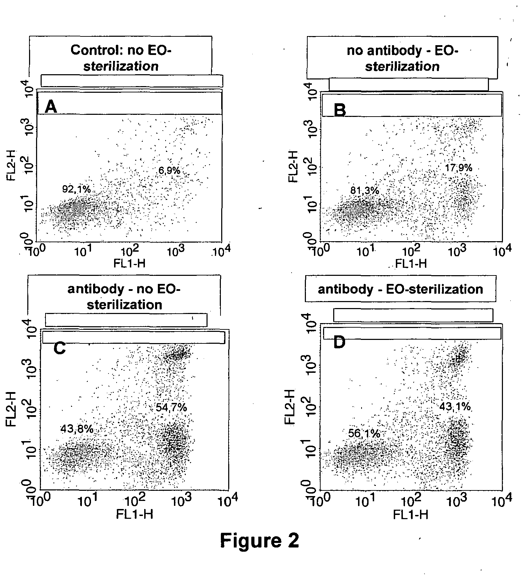

As representatively depicted in Figure 2 the percentage of Jurkat cells undergoing spontanous apoptosis (A) was 6.9 % (range: 6-8 %). Jurkat cells challenged with coated wells that underwent sterilization in the absence of immobilized antibodies (B) exhibited slightly enhanced apoptosis (17.9%; range: 16-20 %). Anti-Fas, immobilized according to the above described coating procedure (C), induced apoptosis in 54.7 % of the cells (range: 54-58 %). After sterilization with ethyleneoxid (EO) the antibody-mediated induction of apoptosis (D) in Jurkat cells was 43.1 % (range: 40-44 %). The EO-mediated reduction of antibody function thus was approximately 25% compared to the unsterilized control (procedure without EO gas). Dot blot analyses (Figure 2) show the fluorescence distribution for the respective cell population (FL1 = apoptosis; FL2 = necrosis).

**Example 2 (for comparison only):**

**Production of a Leukocytes Inhibition Module (LIM)**

For producing a LIM, a foam was soaked in a mixture of 98% methanol and 2% (3-glycidyl-oxypropyl)trimethoxysilane (Sigma) for 20 minutes and then dried, followed by an incubation of 100μg antibody in a buffer consisting of 15mM sodium carbonate and 35mM sodium hydrogencarbonate in water (ph 9) for 2 h at 37°C. Alternatively, PBS can be used as buffer for the antibody. After that, addition of isotonic NaCl solution including 2% human albumin protein concentration is done with another 1 h incubation. Subsequently 10 washing cycles using isotonic sodiumchloride-solution containing 1% human albumin are performed. At least, incubation with isotonic sodium-solution containing 5% human albumin and 5% mannitol for 30 min is followed by drying. Ethylenoxide-sterilisation diminished the antibody activity (tested with Jurkat cells) for about 60 %. After β-radiation using an electron accelerator with 10 MeV with a dose of 25 kGray the antibody activity was preserved to about 70 % compared to the activity without sterilization. Alternatively, the sterilization may be effected using γ-radiation, e.g. using a Co60 source.

A scheme of a LIM is depicted in figure 4.

**Claims**

1. A method of producing a solid coated carrier carrying an antibody, consisting of the steps of:

(a) incubating a solid carrier with a solution comprising 0.1 to 10 % (w/w) or (v/v) of at least one suitable coupler, wherein the coupler is selected from diaminoalkanes, dicarboxylic acids or bi- or multi functionalized polyethyleneglycol, and subsequently removing the solution, thereby attaching a layer of the at least one suitable coupler to the carrier, and, optionally, further comprising a step (a') drying the carrier until the residual content of the solution is less than 10 % of the originally applied solution;
(b) attaching the antibody to the layer of the at least one suitable coupler of step (a) attached to the carrier by incubating the carrier with a preferably buffered aqueous solution containing the antibody and subsequently removing the aqueous solution, further comprising a step (b') subsequent to the step (b) and previous to step (c):
(b') incubating the carrier in a buffered aqueous solution containing a blocking agent and removing the aqueous solution;
or optionally further comprising a step (b") subsequent to the step (b) and previous to step (c):
(b") blocking unbound binding sites using an aqueous solution containing 0.5-10 % (w/w) substances selected from the group consisting of (poly)peptides, hydroxyethylstarch (HES), mannitol, sorbitol and polyethyleneglycol (PEG), milk, soya, wheat or egg derived protein and optionally performing one or more washing steps using an aqueous solution after blocking;
(c) incubating the carrier in an aqueous solution comprising one or more substances selected from polypeptides, at least one amino acid selected from the group consisting of glycine, aspartic acid, glutamic acid, proline, arginine, alanine, asparagine, glutamine, ornithine, hydroxyproline, citrulline, inuline, lysine, leucine, isoleucine, serine, valine, threonine, mannitol, or starch or a mixture thereof, such that the antibody is partially or completely covered by said one or more substances; and, optionally, air-drying the carrier until the residual water content is < 20 % (w/w);

wherein steps (a), (b) and (c) are carried out in the above described order; and

(d) sterilizing the produced solid coated carrier.

2. The method according to claim 1, wherein the carrier in step (c) is incubated in an aqueous solution comprising one or more substances selected from the group consisting of albumin, hydroxyethylstarch (HES), and chaperones.

3. The method according to claim 1, wherein the carrier in step (c) is incubated in an ionic liquid.

4. The method according to any one of claims 1 to 3, wherein the material of the carrier is of porous structure.

5. The method according to claim 4, wherein the material of the carrier is **characterized by** an surface/gaseous volume ratio in a range of 30 cm$^{-1}$ to 300 cm$^{-1}$.

6. The method according to claim 4 or 5, wherein the material of the carrier is **characterized by** a material volume ratio uncompressed/compressed in a range of 4 to 40.

7. The method according to claim 5, wherein the material of the carrier has the structure of hollow fiber, preferably **characterized by** a material volume ratio uncompressed/compressed in a range of 1 to 10 and/or the surface/gaseous volume ratio is in a range of 200 cm$^{-1}$ to 2000 cm$^{-1}$.

8. The method according to any one of claims 1 to 7, wherein the material of the carrier is selected from the group consisting of glass, polyurethane, polyester, polysulfone, polyethylene, polypropylene, polyacryl, polyacrylnitril, polyamid, PMMA, fleece wadding, open porous foam plastic or glass and reticular plastic or glass and structures derived from marine sponges (porifera).

9. The method according to any one of claims 1 to 8, wherein the solution in step (a) is an aqueous solution.

10. The method according to any one of claims 1 to 8, wherein the solution in step (a) is a non-aqueous solution.

11. The method according to claims 1 to 10, wherein the antibody is an antibody fragment, scFv fragment or single domain antibody.

12. The method according to claim 11, wherein the antibody is a monoclonal antibody.

13. The method according to claims 1 to 12, wherein the antibody is of the IgG, IgY or IgM class.

14. The method according to of any one of claims 1 to 13, wherein the antibody is attached to said carrier in step (b) via a covalent bond.

15. The method according to any one of claims 1 to 14, wherein the steps (a) to (c) are effected in a system of rotating tubes that contain the carrier.

16. The method according to claim 15, wherein the solutions are rotated in the system of tubes via a pump.

17. The method according to any one of claims 8 to 16, wherein the carrier made of a material having a porous structure comprises at least one further material.

18. The method according to claim 17, wherein the at least one further material is selected from the group consisting of carbon, SiO$_2$, HES and biotin.

19. The method according to any one of claim 1 to 18, wherein the solution in steps (a) to (c) is an aqueous solution comprising 0.5 to 10 % albumin (v/v) and 0.5 to 5 % mannitol (v/v).

20. The method according to any one of claims 1 to 19, wherein the sterilization of the carrier is effected by ethyleneoxid (EO), beta radiation, gamma radiation, X-ray, heat inactivation, autoclaving or plasma sterilization.

21. The method according to claim 20, further comprising a step (f) of washing the coated carrier after step (d).

22. Use of a solid coated carrier produced or producible by the method of any one of claims 1 to 21 for the preparation of a medical product for the contacting, filtering or cleaning of blood, lymph or liquor cerebrospinal of a patient.

23. An *in vitro* or *ex vivo* method for the manipulation of the composition of blood, lymph or liquor cerebrospinal of a patient comprising the contacting of the blood, lymph or liquor cerebrospinal with a solid coated carrier produced or producible by the method of any one of claims 1 to 21.

24. The use according to claim 22 or the method according to claim 23, wherein the manipulation of the composition of blood, lymph or liquor cerebrospinal of a patient is effected in a batch container which contains the solid coated carrier.

25. The use according to claim 22 or the method according to claim 23, wherein the manipulation of the composition of blood, lymph or liquor cerebrospinal of a patient is effected in a flow through container which contains the solid coated carrier.

26. A method for the diagnosis of a disease comprising the steps of:

(a) contacting body liquid obtained from a patient with a solid coated carrier produced or producible by the method of any one of claims 1 to 21 into which an antibody is embedded under suitable conditions for a specific binding of a pathogen or marker protein indicative for the disease to the embedded antibody; and
(b) detecting whether the pathogen or marker protein indicative for the disease has been bound to the embedded antibody.

27. A method of purifying a compound comprising contacting a mixture comprising the compound to be purified with a solid coated carrier produced or producible by the method of any one of claims 1 to 21.


**Patentansprüche**

1. Verfahren zur Herstellung eines festen beschichteten Trägers der einen Antikörper trägt, bestehend aus den Schritten:

(a) Inkubation eines festen Trägers mit einer Lösung, umfassend 0,1 bis 10 Gew-% oder Vol-% mindestens eines geeigneten Kopplers, wobei der Koppler ausgewählt ist aus Diaminoalkanen, Dicarbonsäuren und bi- oder multi-funktionalisiertem Polyethylenglycol und dem anschließenden Entfernen der Lösung, wodurch eine Schicht des mindestens einen geeigneten Kopplers an den Träger angeheftet wird und, gegebenenfalls, weiterhin umfassend einen Schritt (a') Trocknen des Trägers, bis der restliche Inhalt der Lösung weniger als 10% der ursprünglich eingesetzten Lösung ist;
(b) Anheften des Antikörpers an die Schicht des mindestens einen geeigneten Kopplers aus Schritt (a), die an den Träger anhaftet, durch die Inkubation des Trägers mit einer bevorzugt gepufferten wässrigen Lösung, die den Antikörper enthält, und dem anschließenden Entfernen der wässrigen Lösung, weiterhin umfassend einen Schritt (b') nach dem Schritt (b) und vor dem Schritt (c):

(b') Inkubation des Trägers mit einer gepufferten wässrigen Lösung, die ein Blockierungsagens enthält, und dem Entfernen der wässrigen Lösung;

oder gegebenenfalls weiterhin umfassend einen Schritt (b") nach dem Schritt (b) und vor dem Schritt (c):

(b") Blockierung ungebundener Stellen unter Verwendung einer wässrigen Lösung, die 0,5-10 Gew-% Substanzen enthält ausgewählt aus der Gruppe bestehend aus (Poly)peptiden, Hydroxyethylstärke (HES), Mannitol, Sorbitol und Polyethylenglycol (PEG), Milch, Soja, von Weizen oder Ei stammenden Proteinen und gegebenenfalls Durchführung eines oder mehrerer Waschschritte unter Verwendung einer wässrigen Lösung nach der Blockierung;

(c) Inkubation des Trägers in einer wässrigen Lösung, umfassend eine oder mehrere Substanzen ausgewählt aus Polypeptiden, mindestens einer Aminosäure ausgewählt aus der Gruppe bestehend aus Glycin, Asparaginsäure, Glutaminsäure, Prolin, Arginin, Alanin, Asparagin, Glutamin, Ornithin, Hydroxyprolin, Citrullin, Inulin, Lysin, Leucin, Isoleucin, Serin, Valin, Threonin, Mannitol oder Stärke oder einem Gemisch davon, so dass der Antikörper teilweise oder vollständig von den einen oder mehreren der Substanzen bedeckt ist und, gegebenenfalls, Lufttrocknen des Trägers bis der restliche Wassergehalt < 20 Gew-% ist;
wobei die Schritte (a), (b), (c) in oben beschriebener Reihenfolge ausgeführt werden; und
(d) Sterilisieren des hergestellten festen beschichteten Trägers.

2. Verfahren nach Anspruch 1, wobei der Träger in Schritt (c) in einer wässrigen Lösung inkubiert wird, die eine oder mehrere Substanzen ausgewählt aus Albumin, Hydroxyethylstärke (HES) und Chaperonen umfasst.

3. Verfahren nach Anspruch 1, wobei der Träger in Schritt (c) in einer ionischen Lösung inkubiert wird.

4. Verfahren nach einem der Ansprüche 1 bis 3, wobei das Material des Trägers eine poröse Struktur hat.

5. Verfahren nach Anspruch 4, wobei das Material des Trägers durch ein Oberfläche-/Gasvolumen-Verhältnis im Bereich von 30 cm$^{-1}$ bis 300 cm$^{-1}$ gekennzeichnet ist.

6. Verfahren nach Anspruch 4 oder 5, wobei das Material des Trägers durch ein Materialvolumenverhältnis nicht komprimiert/komprimiert im Bereich von 4 bis 40 gekennzeichnet ist.

7. Verfahren nach Anspruch 5, wobei das Material des Trägers die Struktur einer Hohlfaser hat, bevorzugt **gekennzeichnet durch** ein Materialvolumenverhältnis nicht komprimiert/komprimiert im Bereich von 1 bis 10 und/oder ein Oberfläche-/Gasvolumen-Verhältnis im Bereich von 200 cm$^{-1}$ bis 2000 cm$^{-1}$.

8. Verfahren nach einem der Ansprüche 1 bis 7, wobei das Material des Trägers ausgewählt ist aus der Gruppe bestehen aus Glas, Polyurethan, Polyester, Polysulfon, Polyethylen, Polypropylen, Polyacryl, Polyacrylnitril, Polyamid, PMMA, Vlies-Watte, offenporiger/s Schaumkunststoff oder Glas und netzförmiges Plastik oder Glas und von marinen Schwämmen (Porifera) abgeleiteten Strukturen.

9. Verfahren nach einem der Ansprüche 1 bis 8, wobei die Lösung im Schritt (a) eine wässrige Lösung ist.

10. Verfahren nach einem der Ansprüche 1 bis 8, wobei die Lösung im Schritt (a) eine nicht-wässrige Lösung ist.

11. Verfahren nach einem der Ansprüche 1 bis 10, wobei der Antikörper ein Antikörperfragment, scFv-Fragment oder Einzel-Domäne-Antikörper ist.

12. Verfahren nach Anspruch 11, wobei der Antikörper ein monoklonaler Antikörper ist.

13. Verfahren nach einem der Ansprüche 1 bis 12, wobei der Antikörper der IgG-, IgY- oder IgM-Klasse angehört.

14. Verfahren nach einem der Ansprüche 1 bis 13, wobei der Antikörper an den Träger im Schritt (b) durch eine kovalente Binding angeheftet wird.

15. Verfahren nach einem der Ansprüche 1 bis 14, wobei die Schritte (a) bis (c) in einem System mit rotierenden Röhren, die den Träger umfassen, durchgeführt werden.

16. Verfahren nach Anspruch 15, wobei die Lösungen in dem System mit Röhren mit einer Pumpe rotiert werden.

17. Verfahren nach einem der Ansprüche 8 bis 16, wobei der Träger, der aus einem Material mit poröser Struktur hergestellt ist, mindestens ein weiteres Material umfasst.

18. Verfahren nach Anspruch 17, wobei das mindestens eine weitere Material ausgewählt ist aus der Gruppe

bestehend aus Kohlenstoff, $SiO_2$, HES und Biotin.

19. Verfahren nach einem der Ansprüche 1 bis 18, wobei die Lösung in den Schritten (a) bis (c) eine wässrige Lösung ist, die 0,5 bis 10 Vol-% Albumin und 0,5 bis 5 Vol-% Mannitol umfasst.

20. Verfahren nach einem der Ansprüche 1 bis 19, wobei die Sterilisierung des Trägers mit Ethylenoxid (EO), Beta-Strahlung, Gamma-Strahlung, Röntgenstrahlung, Hitzeinaktivierung, Autoklavieren oder Plasmasterilisation bewirkt wird.

21. Verfahren nach Anspruch 20, weiterhin umfassend einen Schritt (f) des Waschens des beschichteten Trägers nach Schritt (d).

22. Verwendung eines festen beschichten Trägers, der nach dem Verfahren nach einem der Ansprüche 1 bis 21 hergestellt ist oder hergestellt werden kann zur Herstellung eines Medizinprodukts zum Kontaktieren mit, Filtrieren von oder Reinigung von Blut, Lymphe oder Liquor cerebrospinalis eines Patienten.

23. *In vitro* oder *ex vivo* Verfahren zur Manipulation der Zusammensetzung von Blut, Lymphe oder Liquor cerebrospinalis eines Patienten umfassend das Inkontaktbringen des Bluts, der Lymphe oder des Liquor cerebrospinalis mit einem festen beschichteten Träger, der nach dem Verfahren nach einem der Ansprüche 1 bis 21 hergestellt ist oder hergestellt werden kann.

24. Verwendung nach Anspruch 22 oder Verfahren nach Anspruch 23, wobei die Manipulation der Zusammensetzung des Bluts, der Lymphe oder des Liquor cerebrospinalis eines Patienten in einem Batch-Container bewirkt wird, der den festen beschichteten Träger beinhaltet.

25. Verwendung nach Anspruch 22 oder Verfahren nach Anspruch 23, wobei die Manipulation der Zusammensetzung des Bluts, der Lymphe oder des Liquor cerebrospinalis eines Patienten in einem Durchfluss-Container bewirkt wird, der den festen beschichteten Träger beinhaltet.

26. Verfahren zur Diagnose einer Krankheit, umfassend die Schritte:

(a) Kontaktieren von aus einem Patienten gewonnener Körperflüssigkeit mit einem festen beschichteten Träger, der nach dem Verfahren nach einem der Ansprüche 1 bis 21 hergestellt ist oder hergestellt werden kann, in den ein Antiköper eingebettet ist und zwar unter geeigneten Bedingungen für die spezifische Bindung eines Pathogens oder Markerproteins, das die Krankheit anzeigt, an den eingebetteten Antikörper; und

(b) Nachweis, ob das Pathogen oder Markerprotein, das die Krankheit anzeigt, an den eingebetteten Antikörper gebunden hat.

27. Verfahren zur Aufreinigung einer Verbindung, umfassend Kontaktieren eines Gemisches, das die aufzureinigende Verbindung umfasst, mit einem festen beschichteten Träger der nach dem Verfahren nach einem der Ansprüche 1 bis 21 hergestellt ist oder hergestellt werden kann.

**Revendications**

1. Méthode de production d'un support revêtu solide portant un anticorps, consistant en les étapes suivantes :

   (a) incubation d'un support solide avec une solution comprenant 0,1 à 10 % (p/p) ou (v/v) d'au moins un agent de couplage convenable, lequel agent de couplage est choisi parmi les diaminoalcanes, les acides dicarboxyliques ou le polyéthylèneglycol bi- ou multi-fonctionnalisé, et ensuite retrait de la solution, moyennant quoi une couche de l'au moins un agent de couplage convenable est attachée au support, et éventuellement en outre comprenant une étape (a') de séchage du support jusqu'à ce que la teneur résiduelle en la solution soit inférieure à 10 % de la solution appliquée à l'origine;
   (b) attachement de l'anticorps à la couche de l'au moins un agent de couplage convenable de l'étape (a) attachée au support par incubation du support avec une solution aqueuse de préférence tamponnée contenant l'anticorps, et ensuite retrait de la solution aqueuse, comprenant en outre une étape (b') suivant l'étape (b) et précédant l'étape (c):
   (b') incubation du support dans une solution aqueuse tamponnée contenant un agent de blocage et retrait de la solution aqueuse;
   ou comprenant en outre éventuellement une étape (b") suivant l'étape (b) et précédant l'étape (c):
   (b") blocage des sites de liaison non liés par utilisation d'une solution aqueuse contenant 0,5 à 10 % (p/p) de substances choisies dans le groupe constitué par les (poly)peptides, l'hydroxyéthyl-amidon (HES), le mannitol, le sorbitol et le polyéthylèneglycol (PEG), les protéines dérivées du lait, du soja, du petit-lait ou de l'œuf, et éventuellement mise en œuvre d'une ou plusieurs étapes de lavage par utilisation d'une solution aqueuse après le blocage;

   (c) incubation du support dans une solution aqueuse comprenant une ou plusieurs substances choisies parmi les polypeptides, au moins un acide aminé choisi dans le groupe constitué par la glycine, l'acide aspartique, l'acide glutamique, la proline, l'arginine, l'alanine, l'asparagine, la glutamine, l'ornithine, l'hydroxyproline, la citrulline, l'inuline, la lysine, la leucine, l'isoleucine, la sérine, la valine, la thréonine, le mannitol et l'amidon, ou leurs mélanges, de façon que l'anticorps soit partiellement ou complètement recouvert par lesdites une ou plusieurs substances ; et éventuellement séchage à l'air du support jusqu'à ce que la teneur en eau résiduelle soit < 20 % (p/p);
   dans laquelle les étapes (a), (b) et (c) sont effectuées dans l'ordre décrit ci-dessus; et
   (d) stérilisation du support revêtu solide produit.

2. Méthode selon la revendication 1, dans laquelle le support dans l'étape (c) est incubé dans une solution aqueuse comprenant une ou plusieurs substances choisies dans le groupe constitué par l'albumine, l'hydroxyéthyl-amidon (HES) et les protéines chaperons.

3. Méthode selon la revendication 1, dans laquelle le support dans l'étape (c) est incubé dans un liquide ionique.

4. Méthode selon l'une quelconque des revendications 1 à 3, dans laquelle le matériau du support a une structure poreuse.

5. Méthode selon la revendication 4, dans laquelle le matériau du support est **caractérisé par** un rapport surface/volume gazeux situé dans la plage allant de 30 cm$^{-1}$ à 300 cm$^{-1}$.

6. Méthode selon la revendication 4 ou 5, dans laquelle le matériau du support est **caractérisé par** un rapport en volume de matériau non comprimé/comprimé situé dans la plage allant de 4 à 40.

7. Méthode selon la revendication 5, dans laquelle le matériau du support a une structure de fibre creuse, de préférence **caractérisée par** un rapport en volume de matériau non comprimé/comprimé situé dans la plage allant de 1 à 10 et/ou un rapport surface/volume gazeux situé dans la plage allant de 200 cm$^{-1}$ à 2 000 cm$^{-1}$.

8. Méthode selon l'une quelconque des revendications 1 à 7, dans laquelle le matériau du support est choisi dans le groupe constitué par le verre, le polyuréthane, le polyester, la polysulfone, le polyéthylène, le polypropylène, le polyacrylate, le polyacrylonitrile, le polyamide, le PMMA, la ouate non tissée, le verre ou le plastique expansé à pores ouverts et le verre ou le plastique réticulaire, et les structures dérivées d'épongés de mer (porifera).

9. Méthode selon l'une quelconque des revendications 1 à 8, dans laquelle la solution dans l'étape (a) est une solution aqueuse.

10. Méthode selon l'une quelconque des revendications 1 à 8, dans laquelle la solution dans l'étape (a) est une solution non aqueuse.

11. Méthode selon l'une quelconque des revendications 1 à 10, dans laquelle l'anticorps est un fragment d'anticorps, un fragment scFv ou un anticorps à un seul domaine.

12. Méthode selon la revendication 11, dans laquelle l'anticorps est un anticorps monoclonal.

13. Méthode selon l'une quelconque des revendications 1 à 12, dans laquelle l'anticorps est de la classe IgG, IgY ou IgM.

14. Méthode selon l'une quelconque des revendications 1 à 13, dans laquelle l'anticorps est attaché audit support dans l'étape (b) via une liaison covalente.

15. Méthode selon l'une quelconque des revendications 1 à 14, dans laquelle les étapes (a) à (c) sont effectuées dans un système de tubes rotatifs qui contiennent le support.

16. Méthode selon la revendication 15, dans laquelle les solutions tournent dans le système de tubes via une pompe.

17. Méthode selon l'une quelconque des revendications 8 à 16, dans laquelle le support fait d'un matériau ayant une structure poreuse comprend au moins un autre matériau.

18. Méthode selon la revendication 17, dans laquelle l'au moins un autre matériau est choisi dans le groupe constitué par le carbone, $SiO_2$, HES et la biotine.

19. Méthode selon l'une quelconque des revendications 1 à 18, dans laquelle la solution dans les étapes (a) à (c) est une solution aqueuse comprenant 0,5 à 10 % (v/v) d'albumine et 0,5 à 5 % (v/v) de mannitol.

20. Méthode selon l'une quelconque des revendications 1 à 19, dans laquelle la stérilisation du support est effectuée au moyen d'oxyde d'éthylène (EO), d'un rayonnement bêta, d'un rayonnement gamma, de rayons X, d'une inactivation à la chaleur, ou d'une stérilisation en autoclave ou par plasma.

21. Méthode selon la revendication 20, comprenant en outre une étape (f) de lavage du support revêtu après l'étape (d).

22. Utilisation d'un support revêtu solide produit ou pouvant être produit par le procédé de l'une quelconque des revendications 1 à 21 pour la préparation d'un produit médical destiné à venir en contact avec, filtrer ou nettoyer du sang, de la lymphe ou du liquide céphalo-rachidien d'un patient.

23. Méthode *in vitro* ou *ex vivo* pour la manipulation de la composition du sang, de la lymphe ou du liquide céphalo-rachidien d'un patient, comprenant la mise en contact du sang, de la lymphe ou du liquide céphalo-rachidien avec un support revêtu solide produit ou pouvant être produit par la méthode de l'une quelconque des revendications 1 à 21.

24. Utilisation selon la revendication 22 ou méthode selon la revendication 23, dans laquelle la manipulation de la composition du sang, de la lymphe ou du liquide céphalo-rachidien d'un patient est effectuée dans un récipient de lots qui contient le support revêtu solide.

25. Utilisation selon la revendication 22 ou méthode selon la revendication 23, dans laquelle la manipulation de la composition du sang, de la lymphe ou du liquide céphalo-rachidien d'un patient est effectuée dans un récipient à circulation continue qui contient le support revêtu solide.

26. Méthode pour diagnostiquer une maladie, comprenant les étapes suivantes:

(a) mise en contact d'un liquide corporel obtenu auprès d'un patient avec un support revêtu solide produit ou pouvant être produit par la méthode de l'une quelconque des revendications 1 à 21 dans lequel un anticorps est incorporé dans des conditions convenables pour une liaison spécifique d'un pathogène ou d'un marqueur protéique indicatif de la maladie avec l'anticorps incorporé; et
(b) détection que le pathogène ou le marqueur protéique indicatif de la maladie s'est ou non lié à l'anticorps incorporé.

27. Méthode de purification d'un composé, comprenant la mise en contact d'un mélange comprenant le composé devant être purifié avec un support revêtu solide produit ou pouvant être produit par la méthode de l'une quelconque des revendications 1 à 21.

**solid carrier**

Silane-derivate

PEG/albumin/mannitol

Antibody

APC

**Step 1**

**Step 2**

partial coverage

complete coverage

**Step 3**

**Step 4**    **Sterilization**

**Step 5**    **Washing**

partial removal of coverage

complete removal of coverage

**Function**

Figure 1

**Figure 2**

**Figure 3**

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03030949 A

### Non-patent literature cited in the description

- **R. BAMBAUER et al.** *Ther Apher Dial.,* 2003, vol. 7 (4), 382-90
- **G. WALLUKAT et al.** *International Journal of Cardiology,* 1996, vol. 54 (2), 191-195
- **D. GREEN et al.** *Tissue Engineering,* 2003, vol. 9 (6), 1159-1166
- **HARLOW ; LANE.** Antibodies: A Laboratory Manual. Cold Spring Harbor Laboratory Press, 1988
- **HARLOW ; LANE.** Using Antibodies: A Laboratory Manual. Cold Spring Harbor Laboratory Press, 1999
- **SELA.** *Science,* 1969, vol. 166, 1365
- **LAVER.** *Cell,* 1990, vol. 61, 553-6
- **SAMBROOK et al.** Molecular Cloning: A Laboratory Manual. Cold Spring Harbor Laboratory Press, 1989
- MOLECULAR CLONING: A LABORATORY MANUAL. 2001
- **GERHARDT et al.** Methods for General and Molecular Bacteriology. ASM Press, 1994
- **LEFKOVITS.** Immunology Methods Manual: The Comprehensive Sourcebook of Techniques. Academic Press, 1997
- **GOLEMIS.** Protein-Protein Interactions: A Molecular Cloning Manual. Cold Spring Harbor Laboratory Press, 2002
- **SCHOLZ M et al.** *ASAIO J,* 2005, vol. 51, 144-7
- **SCHOLZ M ; CINATL J.** *Med Res Rev,* 2005, vol. 25, 331-42
- **SCHOLZ M et al.** *J Thorac Cardiovasc Surg,* 2004, vol. 127, 1735-42
- **MORENO JB et al.** *Perfusion,* 2004, vol. 19, 11-6
- **SCHUPBACH et al.** *J Aquir Immune Defic Syndr,* 2005, vol. 40, 250-6
- **JUST-NUBLING G et al.** *Infection,* 2003, vol. 31, 318-23